Europäisches Patentamt

European Patent Office

Office européen des brevets

(19)

(11) EP 1 051 983 A1

(12) EUROPEAN PATENT APPLICATION

(43) Date of publication:
**15.11.2000 Bulletin 2000/46**

(51) Int Cl.[7]: **A61L 15/58**, A61L 15/60

(21) Application number: **99122204.3**

(22) Date of filing: **06.11.1999**

(84) Designated Contracting States:
**AT BE CH CY DE DK ES FI FR GB GR IE IT LI LU MC NL PT SE**
Designated Extension States:
**AL LT LV MK RO SI**

(30) Priority: **10.05.1999 EP 99109155**

(71) Applicant: **THE PROCTER & GAMBLE COMPANY Cincinnati, Ohio 45202 (US)**

(72) Inventors:
• **Palumbo, Gianfranco**
  **61348 Bad Homburg (DE)**
• **Preuschen, Judith**
  **55129 Mainz (DE)**

(74) Representative: **Kohol, Sonia et al**
**Procter & Gamble**
**European Service GmbH**
**Sulzbacher Strasse 40-50**
**65824 Schwalbach am Taunus (DE)**

(54) **Hydrogel pressure sensitive adhesives for use as wound dressings**

(57)    The present invention relates to a wound dressing comprising a hydrogel adhesive having improved skin adhesion properties whilst still facilitating easy application and removal from the wearer, and improving the wound healing process.

Figure 1.

EP 1 051 983 A1

**Description**

Field of the Invention

The present invention relates to pressure sensitive hydrogel adhesives for use alone or in combination with medical devices such as wound dressings for the treatment of wounds, cuts, burns, ulcers and the like, on the human body.

Background of the Invention

Bandages, plasters, band aids and the like are well known articles of manufacture that are designed to assist in wound care management. Traditionally such devices comprise an absorbent dressing material which is placed in direct contact with the area to be treated and absorbs the wound exudates, in addition to providing protection against bacteria and dirt thereby minimising the risk of infection. The absorbent dressing material is then typically secured to the wearer. The method used to secure the dressing to the body is to a large extent dependent upon the nature and size of the wound itself. Typically if large skin surfaces are to be treated, the absorbent dressing material is held in place by the use of additional material such as gauze which is wrapped around the absorbent dressing material and the wearer. For smaller treatment areas, the dressing material is typically provided on a support material which is supplied with adhesive for attachment to the wearers' skin adjacent to the area to be treated to form what is commonly known as a plaster.

However the extent to which such plasters can protect and assist the wound during the healing process is severely limited. Infact to a large extent the plaster is only able to provide a protective layer, and absorb the wound exudates. Moreover due to the fact these plasters are typically provided in predetermined strips and can only be utilised to effectively protect wounds of certain dimensions, the applications of such dressings to a variety of types and degree of severity of wounds is limited. In addition a particular problem with such plasters is that the gauze material in addition to absorbing the wound exudates tend to dry out during wear and often becomes adhered to the wound. As a result removal of the plaster is painful not only due to the adhesives but also because the gauze needs to be detached from the site of the healing wound which is both painful and detrimental to the wound healing process and can lead to additional scar formation.

As a consequence the art is replete with references to the improvement of these wound care products. One particular area of development has been in the area of so called hydrogel materials as a replacement for and in addition to the use of traditional absorbent dressing materials. These materials are typically water based three dimensional matrix gels formed by crosslinking polymers, preferably in the presence of a plasticizer. In contrast to the absorbent dressing materials these hydrogel materials are able to absorb some of the wound extrudate whilst not drying out and becoming attached to the wound itself. This is of particular benefit as the hydrogel can then be removed from the wound and if necessary replaced without interfering in the wound healing process or causing any pain.

Such hydrogel materials are disclosed for example in the following publications; US 4 391 278, US 4 593 053, US 4 699 146, US 4 768 523, US 4 909 244, US 5 175 246, US 5 354 790, US 5 780 151, US 5 783 209, EP 793 972, EP 624 635, EP 601 463, EP 506 300, EP 251 774, GB 2 115 431, W098/03208, W098/09666 and WO 98/15245.

In addition to not interfering with the wound healing process the prior art also discloses that certain hydrogel materials also offer a number of additionally desirable benefits. For example hydrogels are typically at least partially transparent thereby allowing the healing process to be observed without having to disturb the wound healing process. The hydrogels also allow a degree of moisture vapour transport so that the normal transport of moisture from the skin is not substantially hindered despite the presence of the hydrogel. Indeed a number of these hydrogel materials also provide a cooling effect which is particularly beneficial in the treatment of burns, but also finds application in minor skin conditions or ailments whereby the itching associated with the later steps of the healing process is reduced. However, the amount of moisture absorbency is not typically controlled such that the wound may still easily become dried out and cause scarring.

Another problem with the hydrogel materials of the prior art is that whilst some of these materials may exhibit a minimal degree of adhesiveness or tackiness this is not sufficient in order to ensure that the hydrogel remains attached to the wound site during normal activities. The wearing conditions of such materials will naturally depend on the nature of the wearer. In any case when the wearer is active, the wearing conditions become much more stressed and the risk of detachment of the hydrogel materials will increase substantially, due to the movement of the wearer and the pressure from the wearer's body and garments. Consequently these hydrogel materials are infact typically supplied or as an extrudable jelly like paste or in a sheet form and have in use little if any resilience and are easily deformed or rubbed off the skin of the wearer. As a result the hydrogel materials are typically supplied with an adhesive supportive substrate to maintain the hydrogel material in place. However these supportive substrates play no role in the wound healing process itself. Moreover the adhesive substrates which are utilised whilst typically having a skin compatible composition are usually strongly adhesive and may cause skin irritation or inflammation. Hence the removal from the skin cannot be achieved without the wearer experiencing pain. This is particularly undesirable under circumstances, where the plaster is misplaced, and removal and reapplication of the plaster once or even a number of times is required.

Another key problem area with conventional plasters which has also not been addressed by the hydrogel materials of the prior art, is the possibility of the wound itself and the area of skin adjacent thereto becoming macerated or infected and inflamed during the period of wear of the plaster, due to the easy access of bacteria for example to the wound site. It would thus be highly desirable to provide a hydrogel adhesive for wound care which adheres only to the skin surface adjacent the periphery of the wound thereby forming a seal around the wound periphery and preventing infection of the wound.

Hence there still exists a need to provide a hydrogel material for use in wound care management which readily adheres to the skin, but preferably not to the wound itself, over extended wearing period without the need for additional traditional type skin adhesives, and which can be removed from the wearer without pain. Moreover there is also a need to provide a hydrogel adhesive material which is capable of absorbing wound extrudates whilst retaining the wound moist and preventing any wound contamination and thereby aiding the wound healing process.

It is yet a further objective of the present invention to provide a hydrogel adhesive will adhere to moist or wet skin. It is yet a further objective to provide a hydrogel adhesive which is provided in an easy and simple form and can be readily utilised by the consumer and cut to size if necessary in order to treat a variety of wound types and sizes.

It has now been surprisingly found that the above drawbacks will be substantially alleviated by a wound dressing comprising a hydrogel adhesive as defined hereinafter. The hydrogel adhesive provides secure attachment to the skin surrounding the wound but not to the wound site itself, is pleasing to the skin upon application, and yet causes no discomfort upon removal. This is achieved by selecting the characteristics of the hydrogel adhesive, particularly in terms of the elastic modulus G' and the viscous modulus G" of the hydrogel adhesive and the thickness C of the layer of hydrogel adhesive applied and the water absorption capability, moisture vapour transport and elasticity of the wound dressing.

### Summary of the Invention

The present invention hence relates to a wound dressing comprising a hydrogel adhesive, said dressing being defined by specific functional parameters. Preferably the wound dressing further comprises a supportive substrate upon which a layer of the hydrogel adhesive according to the invention is applied.

Detailed analysis of the sequence of common situations occurring from the application of a wound dressing to the time of removal of such a dressing has shown that specific adhesive characteristics need to be preferably satisfied in order to achieve certain desirable performance objectives, in particular to secure initial attachment, secure attachment during use and painless removal after wear. The characteristics which have been considered in this context are the elastic modulus describing the elastic behaviour of the material and the viscous modulus which describes the viscous behaviour of the hydrogel adhesive material.

The viscous behaviour of the hydrogel adhesive can be interpreted to represent an indication of the ability of the adhesive to quickly attach and securely adhere to a particular surface. The elastic behaviour can be interpreted as an indication of the "hardness" behaviour of the hydrogel adhesive. Its value is also critical for good initial attachment. Their combination is believed to be an indicator of the required force upon removal. The relation between elastic and viscous modulus is considered to be an indication on which fraction of the removal energy will be dissipated within the hydrogel adhesive and which fraction is available to trigger the actual removal.

In order to provide adhesives for secure initial and prolonged attachment and easy/painless removal the relation between the elastic modulus and the viscous modulus as well as their dynamic behaviour is also of importance.

The hydrogel adhesive has an elastic modulus at a temperature of 37°C (100° Fahrenheit) abbreviated $G'_{37}$, a viscous modulus at a temperature of 37°C (100° Fahrenheit) of $G''_{37}$, and a viscous modulus at a temperature of 25°C (77° Fahrenheit) of $G''_{25}$.

According to the present invention, it has been ascertained that the relation between the thickness or calliper C, measured in millimetres (mm), of the layer in which the hydrogel adhesive is provided, typically onto at least a portion of the supportive substrate or the skin itself, and the viscous modulus $G''_{25}$ at about 100 rad/sec of the hydrogel adhesive, is relevant to the scope of providing an easy and painless removal from the wearer's skin of such a wound dressing.

The hydrogel adhesive of the present invention is provided as a layer having a thickness C such that the viscous modulus $G''_{25}$ (100 rad/sec) and the thickness C satisfy the following empirical equation:

$$G''_{25} \leq [(7.00 + C) \times 3000] \text{ Pa}$$

and preferably also the following empirical equation:

$$G''_{25} \leq [(5.50 + C) \times 1700] \text{ Pa}$$

In addition it is also imperative that the hydrogel adhesive provide an adhesiviness such that the peel force as determined herein is from 0.5N/inch to 6N/inch, preferably from 1.0N/inch to 3.5N/inch, more preferably from 1.0N/inch to 2.5N/inch. Preferably the peel strength should remain constant under normal conditions over the time period of wear.

The hydrogel adhesive according to the present invention preferably also satisfies the following conditions;

$G'_{37}$ (1 rad/sec)      is in the range 500 Pa to 20000 Pa, preferably 700 Pa to 15000 Pa, most preferably 1000 Pa to 10000 Pa.

$G''_{37}$ (1 rad/sec)      is in the range 100 Pa to 15000 Pa, preferably 100 Pa to 10000 Pa, most preferably 300 Pa to 5000 Pa.

and the ratio of $G'_{37}$ (1 rad/sec) / $G''_{37}$ (1 rad/sec) is in the range of 1 to 30.

The rheological behaviour can also be related to the values of the Glass Transition Temperature Tg. For hydrogels adhesives according to the present invention Tg should preferably be less than -15°C, more preferably less than -20°C and most preferably less than -25°C.

Provided the above rheological conditions are satisfied the hydrogel adhesives will also satisfy conditions such as sufficient cohesiveness (to prevent residue of hydrogel adhesive on the skin) which are critical for commercial use of such hydrogel adhesives and apparent to those skilled in the art.

Further analysis of the biological processes which occur during wound healing has also identified other performance criteria which need to be satisfied by the wound dressing in order to promote and preferably accelerate the wound healing process and prevent infection. The characteristics which have been considered in this context are the moisture vapour transport rate, moisture absorption capacity and elasticity.

According to the present invention it is a further essential feature that the wound dressing has a vapour permeability of from $100g/(m^2.24hrs.)$ to $2000g/m^2/24hrs.)$, preferably from $200g/(m^2.24hrs.)$ to $1500g/(m^2/24hrs.)$, more preferably from $200g/(m^2.24hrs.)$ to $1000g/(m^2/24hrs.)$. In addition the wound dressing of the present invention further exhibits a water absorption capacity as defined hereinafter of at least 100% by weight preferably at least 300%, more preferably at least 500% most preferably at 1000% by weight after 48hrs, and a water absorption/transport rate of at least $0.0001g/cm^2/s^{1/2}$, preferably from $0.0005g/cm^2/s^{1/2}$ to $0.1g/cm^2/s^{1/2}$, more preferably from $0.001g/cm^2/s^{1/2}$ to $0.05g/cm^2/s^{1/2}$, most preferably from $0.0075g/cm^2/s^{1/2}$ to $0.035g/cm^2/s^{1/2}$.

Moreover it is also essential that the wound dressing having a flexibility as defined by the test method hereinafter of less than 0.022N, preferably less than 0.012N, most preferably less than 0.0016N provide such a benefit. It is also essential that said wound dressing has a tensile strength at 50% elongation as defined herein of less than 25N, preferably less than 10N.

Hydrogel adhesive compositions which satisfy the above criteria can be used as adhesives for wound dressings provided they also satisfy the common requirements of being safe for use on human or animal skin during use and disposal.

Often the criteria of hygienic appearance such that adhesive compositions which are transparent or white upon application are preferred.

Detailed description of the Invention

According to the present invention the wound dressing comprises as an essential component a hydrogel adhesive as defined herein. The adhesive is preferably provided on the wearer facing surface of a supportive substrate, as a layer having a thickness or calliper C that is preferably constant. The layer is preferably continuous but may alternatively be provided in a discontinuous manner, e.g. in form of dots, spirals, or stripes.

Even though adhesives such as pressure sensitive adhesives are utilised on the human skin, hair and mucous tissues, it is understood that the hydrogel adhesives of the present invention could only with difficulty be considered typical pressure sensitive adhesives (referred to as PSA hereinafter) on the basis of the most characteristic rheological behaviours identifying such materials.

In fact as the person skilled in the art of adhesives knows, the most characteristic feature that distinguishes a PSA from other substances that can temporarily adhere objects (e.g. water between two glass plates could) is the fact that their rheological parameters and especially the Elastic Modulus G' vary greatly with the frequency of applied stresses. More in particular, G' of PSA can increase over some orders of magnitude, while the frequency of applied stresses varies from typical bonding frequency to typical debonding frequency, i.e. 1 rad/s to 100 rad/s as indicated below.

As a first consequence, it is therefore inadmissible to define materials intended for use as "adhesives" by giving values of rheological parameters and especially of G' at a fixed value of frequency. This can be misleading because in the absence of other characteristics it will include materials which have no practical value. It is hence necessary that rheological characterisation must be on the basis of dynamic considerations. This not only applies to the Elastic Modulus G' but also to the viscous modulus G" and hence also for tan (d) = G" / G'.

It is well known that typical PSAs have not only a high variation of G' across the considered frequencies, but also that there is an even higher variation of G" which can get close or become even higher than the value of G', i.e. tan (d) becomes about or even greater than 1, in particular at the frequencies that are typical of debonding.

Without wishing to be bound by theory this can be interpreted as meaning that a high fraction of the energy applied for the debonding is dissipated within the adhesive (so it is not effective in causing the debonding) and through the interface of the adhesive and the skin, while this fact causes macroscopically the recording of a very high level of adhesive force.

As indicated above materials useful as hydrogel adhesives according to the present invention have rheological characteristics which are measured at a reference temperature of $37°C$ (as usual body temperature of humans) and in a range of frequencies. It has been found that upon application of a wound dressing with a hydrogel adhesive, the adhesive contact is formed at a low frequency, while debonding happens at the speed of removing the dressing. This speed is expressed as a frequency of 100 rad/s, while the low frequency of forming the adhesive bond has been found to be on the order of 1 rad/s. Therefore, the frequency range for use according to the present invention is between 1 and 100 rad/s.

In order to provide good conditions of bonding, i.e. at a frequency of about 1 rad/sec, the absolute values of the elastic modulus should not be too high, otherwise the adhesive is too hard and it is not able to intimately join or mold to the surface to which it is expected to adhere. It is also important to have a low absolute value of G" in order to have good cohesion while the material remains soft and capable of gently adhering to skin.

The ratio of $G'_{37}$ (1 rad/sec) over $G''_{37}$ (1 rad/sec) is important to ensure that these two values are balanced upon adhesion to the skin. Importantly, the ratio of $\dfrac{G'_{37}\ (100\ rad/sec) - G''_{37}\ (100\ rad/sec)}{G'_{37}\ (1\ rad/sec) - G''_{37}\ (1\ rad/sec)}$ needs to be large enough to ensure that the dynamic behaviour of both the elastic and the viscous module are maintained in a relationship which provides secure adhesion and painless and easy removal.

Finally the person skilled in the art will also recognise that the Glass Transition Temperature Tg of the adhesive composition, is a parameter which is useful to more fully define the group of useful adhesives.

Hence according to the present invention, the hydrogel adhesive of the present invention is provided as a layer having a thickness C such that the viscous modulus $G''_{25}$ (100 rad/sec) and the thickness C satisfy the following empirical equation:

$$G''_{25} \le [(7.00 + C) \times 3000]\ Pa$$

and preferably also the following empirical equation:

$$G''_{25} \le [(5.50 + C) \times 1700]\ Pa$$

The following set of characteristics should preferably be satisfied for the adhesive of the present invention:

$G'_{37}$ (1 rad/sec)     is in the range 500 Pa to 20000 Pa, preferably 700 Pa to 15000 Pa, most preferably 1000 Pa to 10000 Pa.

$G''_{37}$ (1 rad/sec)     is in the range 100 Pa to 15000 Pa, preferably 100 Pa to 10000 Pa, most preferably 300 Pa to 5000 Pa.

the ratio of $G'_{37}$ (1 rad/sec) / $G''_{37}$ (1 rad/sec) is in the range of 1 to 30.

the ratio $\dfrac{G'_{37}\ (100\ rad/sec) - G''_{37}\ (100\ rad/sec)}{G'_{37}\ (1\ rad/sec) - G''_{37}\ (1\ rad/sec)}$ is not less than 0.5, preferably in the range 0.7 to 10, most preferably in the range 1 to 7.

The value of the ratio $G'_{37}/G''_{37}$ at least for the frequency range from above 1 rad/s up to 100 rad/s should preferably be 3.3 or above, more preferably 5 or above, most preferably 10 or above, while not exceeding about 30, preferably 20, anywhere in the frequency interval.

The rheological behaviour can also be related to the values of the Glass Transition Temperature Tg. For topical adhesives according to the present invention Tg should preferably be less than -15°C, more preferably less than -20°C and most preferably less than -25°C.

In addition to meeting the above rheology parameters it is also essential that the wound dressing of the present invention has an adhesivity such that it has a peel force of from 0.5N/inch to 6N/inch, preferably from 1N/inch to 3.5N/inch, more preferably from 1.0N/inch to 2.5N/inch.

According to the present invention it has now also been surprisingly found that wound dressings which are compliant with the skin and ensure secure attachment and painless removal of the device to the skin must not only meet the above rheology requirements but also a elasticity/flexibility parameter as defined hereinafter.

In particular, it has been found that in order to ensure wearer comfort of such wound dressings and especially in order to allow the wearer of such dressings to undertake normal bodily movements and activities, it is essential that the wound dressing not only provide secure and painless attachment, and removal from the skin, but also that the wound dressing allows the skin bound to the hydrogel adhesive to move unhindered.

Consequently it is therefore essential that the wound dressing exhibit sufficient flexibility to enable the dressing to be applied to the skin at the site of the wound and adapt to the contours of the body. In particular according to the present invention it has been found that the wound dressing having a flexibility as defined by the test method hereinafter of less than 0.022N, preferably less than 0.012N, most preferably less than 0.0016N provide such a benefit. Moreover, it is also particularly beneficial if the wound dressing skin is selected such that it allows the skin to stretch as required by certain bodily movements or functions without the wearer experiencing any pain but again still ensuring secure attachment. In particular it has been found that the ability of the wound dressing to stretch with the skin can be quantified by the tensile strength of the wound dressing as detailed in the test method hereinafter. Hence, in a particularly preferred embodiment said wound dressing also has a tensile strength at 50% elongation as defined herein of less than 25N, preferably less than 10N. Whilst not being bound by theory it is further believed that the ability of the wound dressing to flex and stretch is dependent upon the combination of the properties of the substrate material if present and in particular the elastic modulus (G') of the hydrogel adhesive.

In a particularly preferred embodiment the wound dressing should also exhibit compressibility. In other words, if the wound dressing is applied to stretched skin for example or if the skin becomes stretched during wear, the skin bonded to the adhesive should be able to contract once the wearer resumes a relaxed position without the wearer experiencing any pain and whilst preventing the skin from buckling and forming channels through the hydrogel adhesive.

In order to provide wound healing it is important to recognise the different stages which occur and the required environment at a particular stage to promote rapid healing. The wound healing process can be divided into three key phases; I Inflammatory Phase, II Proliferation Phase and III Remodelling Phase. The inflammatory phase occurs immediately and up to about 5 days during which the blood clot is formed. The proliferation phase which typically lasts up to 3 weeks, involves granulation, whereby fibroblasts lay a bed of collagen, contraction of the wound edges and epithelialization. The final remodelling phase occurs from 3 weeks to 2 years during which period new collagen is formed.

In particular during the inflammatory phase it is important that the wound dressing does not hinder the normal healthy skin processes or the would healing processes. Thus according to the present invention it is also essential that the wound dressing has a vapour permeability of from $100g/(m^2/24hrs.)$ to $2000\ g/(m^2.24hrs.)$, preferably from $200g/m^2/24hrs.)$ to $1500g/(m^2/24hrs.)$, more preferably from $200g/(m^2/24hrs.)$ to $1000g/m^2/24hrs$. By ensuring that the wound dressing is moisture vapour permeable the surface area of skin covered by the wound dressing is able to function normally so that body temperature cooling mechanisms are not substantially hindered and the creation of an occlusive environment at the surface of the wound site is hindered.

In addition, another essential feature of the present invention is the ability of the wound dressing to absorb moisture. This is important so that the fluids which are dissipated from the wound particularly immediately after the formation of the wound are absorbed by the dressing so as to avoid inflammation or skin maceration. On the other hand it has also been recognised that the maintenance of the wound in a moist environment is beneficial to the wound healing process and stimulates healing and reduces infection rates. Thus it is also important to prevent the wound becoming dry. In addition whilst not being bound by theory it is believed that the ability of the hydrogel adhesive to absorb fluid also plays an important role in the generation of a seal around the periphery of the wound site. The presence of the wound exudate causes a thin layer of fluid to be formed over the wound site and this prevents the hydrogel adhesive from adhering to the wound. However in the absence of such fluid i.e. at the periphery of the wound the hydrogel adhesive maintains its adhesiviness. As a result the hydrogel adhesives of the present invention result in site specific adhesion as required. It has been identified that a wound dressing having a moisture absorption capacity of at least 100% by weight, preferably at least 300%, more preferably at least 500% most preferably at least 1000% in 48 hrs and, which has a water absorption transport rate of at least $0.0001g/cm^2/s^{1/2}$, preferably from $0.0005g/cm^2/s^{1/2}$ to $0.1g/cm^2/s^{1/2}$, more preferably from $0.001g/cm^2/s^{1/2}$ to $0.05g/cm^2/s^{1/2}$, most preferably from $0.0075g/cm^2/s^{1/2}$ to $0.035g/cm^2/s^{1/2}$ will result in the required absorption and wound sealing.

According to the present invention any medically suitable substantially water insoluble pressure sensitive hydrogel adhesive meeting the above parameters may be usefully employed in the present invention. The term hydrogel adhesive as used herein refers to a material comprising a polymer which forms a 3-dimensional matrix, and comprises less than 10%, preferably less than 5% by weight of said adhesive of hydrocolloid particles.

The term hydrocolloid particles as used herein refers to materials mixtures of materials selected from starch, modified starches such as dextrin, cellulose ester such as carboxymethycellulose, and natural gums such as pectin karaya, gelatin, guar gum, gum arabic, locust bean gum, and carboxypolymethylene.

In order to provide adhesive compositions which preferably satisfy the requirements of the above rheological and physical characteristics of an adhesive any medically suitable substantially water insoluble pressure sensitive adhesives comprising a polymer which forms a 3-dimensional matrix preferably meeting the these characteristics may be utilised.

According to the present invention the 3 dimensional matrix also referred to herein as a gel, comprises as an essential component a polymer which can be physically or chemically cross linked. The polymer may be naturally or synthetically derived. The uncrosslinked polymer includes repeating units or monomers derived from vinyl alcohols, vinyl ethers and their copolymers, carboxy vinyl monomer, vinyl ester monomers, esters of carboxy vinyl monomers, vinyl amide monomers, anionic vinyl monomers, hydroxy vinyl monomers, cationic vinyl monomers containing amines or quaternary groups, N-vinyl lactam monomer, polyethylene oxides, polyvinylpyrrolidone (PVP), polyurethanes, acrylics such as methyl acrylate, 2-hydroxyethyl methacrylate, methoxydiethoxyethyl methacrylate and hydroxydiethoxyethyl methacrylate, acrylamides, and sulphonated polymers such as acrylamide sulphonated polymers for example 2 acrylamido methylpropane sulphonic acid and acrylic (3-sulphopropyl) ester acid, and mixtures thereof. Also acrylonitrile, methacrylamide, N,N,-dimethylacrylamide, acrylic esters such as methyl, ethyl and butyl acrylates. Alternatively, the uncrosslinked polymer may be a homopolymer or copolymer of a polyvinyl ether, or a copolymer derived from a half ester of maleic ester. Similarly any other compatible polymer monomer units may be used as copolymers such as for example polyvinyl alcohol and polyacrylic acid or ethylene and vinyl acetate.

As another alternative, the polymers may be block copolymer thermoplastic elastomers such as ABA block copolymers such as styrene-olefin-styrene block copolymers or ethylene-propylene block copolymers. More preferably such polymers include hydrogenated grade styrol/ethylene-butylene/styrol (SEBS), styrene/isoprene/styrene (SIS), and styrol/ethylene-propylene/styrol (SEPS).

Particularly preferred polymers are acrylics, sulphonated polymers such as acrylamide sulphonated polymers, vinyl alcohols, vinyl pyrrolidone, polyethylene oxide and mixtures thereof. Most preferred are nitrogen containing polymers.

According to the present invention the 3 dimensional adhesive matrix also essentially comprises a plasticiser, which is preferably a liquid at room temperature. This material is selected such that the polymer may be solubilized or dispersed within the plasticiser. For embodiments wherein irradiation cross linking is to be carried out, the plasticiser must also be irradiation cross linking compatible such that it does not inhibit the irradiation cross linking process of the polymer. The plasticiser may be hydrophilic or hydrophobic.

Suitable plasticisers include water, alcohols, polyhydric alcohols such as glycerol and sorbitol, and glycols and ether glycols such as mono- or diethers of polyalkylene gylcol, mono- or diester polyalkylene glycols, polyethylene glycols (typically up to a molecular weight of about 600), glycolates, glycerol, sorbitan esters, esters of citric and tartaric acid, imidazoline derived amphoteric surfactants, lactams, amides, polyamides, quaternary ammonium compounds, esters such phthalates, adipates, stearates, palmitates, sebacates, or myristates, and combinations thereof. Particularly preferred are polyhydric alcohols, polyethylene glycol (with a molecular weight up to about 600), glycerol, sorbitol, water and mixtures thereof.

Typically the adhesive comprises a ratio of polymer to plasticiser by weight of from 1:100 to 100:1, more preferably from 50:1 to 1:50. However, the exact amounts and ratios of the polymer and plasticiser will depend to a large extent on the exact nature of polymer and plasticisers utilised and can be readily selected by the skilled person in the art. For example a high molecular weight polymer material will require a greater amount of plasticiser than a low molecular weight polymer.

Other common additives known in the art such as preservatives, antioxidants, pigments, mineral fillers and mixtures thereof may also be comprised within the adhesive composition in quantities up to 10% by weight each respectively.

According to the present invention the polymer component of the adhesive can be physically or chemically cross linked in order to form the 3-dimensional matrix. Physical cross linking refers to polymers having cross links which are not chemical covalent bonds but are of a physical nature such that there are areas in the 3-dimensional matrix having high crystallinity or areas having a high glass transition temperature. Chemical cross linking refers to polymers which are linked by chemical bonds. Preferably the polymer is chemically cross linked by radiation techniques such as thermal-, E beam-, UV-, gamma or micro-wave radiation.

In addition when chemical crosslinks are formed in the system, a polyfunctional cross linker and/or a free radical initiator may be present in the premix to initiate the crosslinking upon irradiation. Such an initiator can be present in quantities up to 5% by weight, preferably from 0.02% to 2%, more preferably from 0.02% to 0.2%. Suitable photoinitiators include the Irgocure series available from Ciba Speciality Chemicals. In addition from 0.02% to 2% of thermal initiators may also be used.

The resulting adhesive composition is mainly hydrophilic. Hydrophobic and mixed phase compositions are dependant upon the nature of the components of the adhesive. In addition a mixture of monomers whether hydrophilic or both hydrophobic and hydrophilic may result in a single phase or mixed phase of at least 2 phases. Preferably, the adhesives of the present invention are mixed phase hydrophilic hydrophobic.

A mixture of monomers which may result in 1, 2 or more phases are preferred. Mixed phase adhesives are compositions in which both hydrophobic and hydrophilic components, preferably in both plasticisers and polymers, form two or more separate phases. In such cases an emulsifier is preferably present at a suitable level to form stable emulsions between the incompatible phases.

The adhesive is provided, typically on at least a portion of the wearer facing surface of a supportive substrate, as a layer having a thickness or calliper C that is preferably constant, or that alternatively can vary over the surface of application of the adhesive.

When considering particularly the removal phase of an adhesive composition for attachment to the skin of a wearer, it is commonly recognised that good conditions of removal, i.e. at a frequency of about 100 rad/sec, of the adhesive applied to at least part of the wearer facing surface of the wound dressing, are achieved when the adhesive can be easily removed from the skin, and particularly from the bodily hair that may be located on this area of the skin, where the wound dressing contacts the body, without causing pain to the wearer, therefore without adhering too hard upon removal, to the skin and the hair of the wearer. Moreover, a good removal implies that the adhesive does not leave residues on the skin or on the hair.

The relationship between the thickness or calliper C measured in millimetres (mm) of the layer of the adhesive typically onto at least part of the wearer's facing surface of the substrate, and the viscous modulus $G''_{25}$ at 25°C at about 100 rad/sec of the topical adhesive gives an indication of painless and easy removal of the adhesive from the skin.

Without being bound to any theory, it is believed that for higher values of $G''_{25}$ at 100 rad/sec, which overall correspond to a higher adhesiveness of the composition, a thicker calliper or thickness C of the adhesive layer is needed so that the energy applied for the removal is more evenly distributed within the mass of the adhesive, and is therefore transferred smoothly to the skin, so avoiding peaks of energy that typically cause the pain sensation to the wearer. In other words, thinner layers of the adhesive necessitate an adhesive with a lower $G''_{25}$ at 100 rad/sec to achieve a reduced pain sensation upon removal of the device.

According to the present invention, the adhesive is preferably provided as a layer having a thickness C such that the viscous modulus $G''_{25}$ (100 rad/sec) and the thickness C of the adhesive layer satisfy the following empirical equation:

$$G''_{25} \leq [(7.00 + C) \times 3000]\ Pa$$

and preferably the following empirical equation:

$$G''_{25} \leq [(5.50 + C) \times 1700]\ Pa$$

While in a preferred embodiment of the present invention the thickness C of the adhesive layer is constant, such an adhesive layer can also have different thicknesses in different portions, provided that the above mentioned relationship between C and $G''_{25}$ is in any case satisfied in each portion.

In order to protect the hydrogel adhesive, prior to use it typically covered with a release paper such as siliconized paper. The hydrogel adhesive can cover the entire wearer facing surface of the supportive substrate but may also be provided with at least one, or preferably two non-adhesive portions. These portions can thereby serve to facilitate the placement and removal of the wound dressing whilst avoiding contact with the hydrogel adhesive and thereby preventing any contamination. These portions are however preferably also covered by the release means. Before application to the skin of the wearer, the release means if present is removed.

Typically, the hydrogel is provided as a layer at a thickness of from 0.02 mm to 5 mm, preferably from 0.5 mm to 4.0 mm, more preferably from 1 mm to 3 mm. Typically the hydrogel adhesive is applied at a basis weight of from

$20g/m^2$ to $2500g/m^2$, more preferably from $500g/m^2$ to $2000g/m^2$ most preferably from $700g/m^2$ to $1500g/m^2$ depending on the end use envisioned. For example, for large surfaces the amount of adhesive may be less than for wound dressings designed for small applications.

According to the present invention the wound dressing in addition to the hydrogel adhesive preferably further comprises a supportive substrate to render one surface of the hydrogel adhesive that may come into contact with garments of the wearer, non adhesive and to provide strength and resiliency. The substrate material must be selected so as to ensure that the required parameters discussed hereinabove are obtained. In the absence of a supportive substrate material the hydrogel adhesive composition is typically treated so as to render the garment facing surface of the hydrogel adhesive substantially non adhesive.

The supportive substrate may be selected from any suitable material and should preferably be selected such that it is made of soft, flexible and malleable material to allow easy placement of the wound dressing to the skin. Any materials which meet the parameter requirements of the wound dressing may be usefully employed herein. Typical materials include nonwoven materials, wovens, open celled thermoplastic foams, composites of open celled foams and stretch nonwoven, and or breathable films and any combination thereof. Preferably, suitable substrates have an overall thickness within the general range of 0.1 to 5 millimetres and a density of 5 to 250 $g/m^2$, more preferably 50 $g/m^2$. Other thermoplastic foam materials, or other suitable plastics sheet materials having the described properties of such foams (i.e., softness, pliability, stretchability, and contractability) might also be used. Preferably the substrate comprises an open celled foam, a nonwoven or a combination thereof. Open celled foams have been found particularly advantageous as the hydrogel adhesive can embed itself therein and thereby prevents delamination.

The substrate may comprise a number of layers such as non-wovens or films. In a preferred embodiment of the present invention a laminate may be formed from a foam or non-woven layer and a film. The laminate can be formed by means known to the man skilled in the art.

Any non-woven layer can comprise felt fabrics, spunlaced fabrics, fluid jet entangled fabrics, air-laid fabrics, wet-laid fabrics, dry-laid fabrics, melt-blown fabrics, staple fibre carding fabrics, spunbonded fabrics, stitch-bonded fabrics, apertured fabrics, combinations of the above or the like.

Suitable film materials for any of said layers preferably comprise a thermoplastic material. The thermoplastic material can be selected from among all types of hot-melt adhesives, polyolefins especially polyethylene, polypropylene, amorphous polyolefins, and the like; material containing meltable components comprising fibres or polymeric binders including natural fibres such as cellulose - wood pulp, cotton, jute, hemp; synthetic fibres such as fibreglass, rayon, polyester, polyolefin, acrylic, polyamid, aramid, polytetrafluroethylene metal, polyimide; binders such as bicomponent high melt/low melt polymer, copolymer polyester, polyvinyl chloride, polyvinyl acetate/chloride copolymer, copolymer polyamide, materials comprising blends wherein some of the constituent materials are not meltable; air and vapour permeable materials including microporous films such as those supplied by EXXON Chemical Co., III, US under the designation EXXAIRE or those supplied by Mitsui Toatsu Co., Japan under the designation ESPOIR NO; and monolithic breathable materials such as Hytrel™ available from DuPont and Pebax™ available from ELF Atochem, France. Film materials which are not inherently moisture vapour permeable will require modification such as the provision of apertures in order to ensure that the wound dressing material meets the required moisture permeability requirements.

A particularly preferred substrate is a laminate of Pebax™ MV3000 at 300μm and a nonwoven (Fibrella 2000, at $30g/m^2$).

The supportive substrate can also be treated with agents to improve its the tactile perceivable softness. The agents include but are not limited to vegetable, animal or synthetic oils, silicone oils and the like. The presence of these agents are known to impart a silky or flannel-like feel to the substrate without rendering it greasy or oily to the tactile sense of the wearer. Additionally, surfactant material, including anionic, non-ionic, cationic and amphoteric surfactants, may be added to further enhance softness and surface smoothness.

The hydrogel adhesive can be applied to the supportive substrate by any means known in the art such as slot coating, spiral, or bead application or printing, typically prior to the curing step. Typically the entire wearer facing surface of the supportive substrate is covered with the hydrogel adhesive of the present invention. In this manner the wound dressing can be supplied in large strips or rolls which can be readily cut to size by the consumer to meet the particular needs of the wound.

**Test methods**

Vapour Permeability Test

The Vapour permeability test is utilised to quantify the vapour transmission properties of the wound dressing.

**Basic Principle of the Methods**

The basic principle of the test is to quantify the extent of water vapour transmission through the wound dressing. The test method that is applied is based on a standardised textile industry applied test method and commonly referred to as the "cup test method". The test is performed in a stable temperature/humidity laboratory maintained at a temperature of 23° C at 50% RH for a period of 24 hours.

**Apparatus**

1) Sample cup (Diameter 6 cm)
2) Syringe to introduce the distilled water into the completed sample cup.
3) Wax to seal the cup once sample has been arranged.
4) A circular punch to facilitate preparation circular samples of diameter = 30 mm.
5) Laboratory of stable climatic conditions (23° C ± 0.5°C / 50% RH ± 1 % RH)
6) Laboratory balance accurate to 4 decimal places.

**Sample Preparation / Measurements:**

A representative wound dressing is selected and a sample is cut to size using the punch. The sample cut is sufficiently large to adequately overlap the sample holder and to ensure material that may have been damaged or undesirably stretched due to the cutting operation lies outside of the measurement centre when the measurement is performed. The sample is so arranged onto of the sample cup so as to fully overlap the cup. The sample is oriented so as to ensure that the surface exposed to the laboratory environment is the same that would be found while wearing the dressing.

The closure ring of the sample cup is then placed onto the sample and pushed down. This ensures that the excess material is held firmly in place and does not interfere with the measurement. A wax is then applied to the entire surface of the closure ring to ensure the whole upper part of the apparatus is closed to the environment. Distilled water (5 ± 0.25 ml) is introduced with the syringe into the sealed sample cup via the minute perforation. Finally this perforation is sealed with silicone grease.

The entire cup (containing sample and water) is weighed and the weight recorded to 4 decimal places. The cup is then placed in a ventilation stream generated by a fan. The air flowing over the top of the sample cup is 3 ± 0.3 m/sec and confirmed via a wind velocity meter ("Anemo", supplied by Deuta SpA., Italy). The sample cup remains in the ventilated test field for a period of 24 hrs and is then re-weighted. During this period if the test sample is sufficiently breathable the liquid in the sample holder is able to diffuse out of the sample holder and into the laboratory environment. This results in a reduction in the weight of water in the sample holder that can be quantified on re-weighing the complete sample cup following the 24 hr period.

The vapour permeability value is determined as the weight loss divided by the open area of the sample holder and quoted per day.

$$\text{i.e. Vapour Permeability} = \text{Weight Loss (g)} / (0.003 \, \text{m}^2/24 \, \text{hrs})$$

**Flexibility test method**

The following test method is utilised to determine the flexibility or stiffness of samples of the wound dressing when the sample is compressed in the MD.

In principle the test method measures the average force required to compress/fold the sample in MD at a force range of 0.001 to 0.3 Newtons.

Equipment:

Tensile Tester: Instron (Mod: 6021)

Scissors

Tensile Tester setting:

1. Calibrate the Load Cell (1 Newton).
2. Set the Tensile Tester to run a Compression test.
3. Set clamp distance at 50mm.
4. Set test speed at 100mm/minute.
5. Set the dimension of the deformation at 35mm.
6. Set the Tensile Tester to acquire Average Load (Newton).

Sample Preparation:

1. Test samples are prepared by cutting using scissors 3 samples of dimensions (6x2 cm in the MDxCD direction).
2. If present release paper is removed from the samples.

Test operation:

1. Tensile tester setting
2. Place the samples between the clamps placing them symmetrically with to the clamps.
3. Run the test on a minimum of 3 samples.
4. Report the average force of 3 tests in N (Newton).

The test sample needs to bend during the test so as a form a C like fold in the centre of the sample. Tests in which the sample does not fold in this manner but adopt other configurations such as S-like folds are to be ignored and the test repeated.

**Tensile strength test**

The following test method is utilized to determine the elongation of samples of the wound dressing when the sample is stretched in the MD.
In principle the test method measures the average force required to stretch the sample by 50%, 100% and to break the sample.

Equipment:

Tensile Tester: Instron (Mod: 6021)

Tensile Tester setting:

1. Calibrate the Load Cell (10 Newton).
2. Set the Tensile Tester to run a tension test.
3. Set clamp distance at 40mm.
4. Set test speed at 100mm/minute.
5. Set the Tensile Tester to acquire Average Load (Newton).

Sample Preparation:

1. Test samples are prepared by cutting using scissors 3 samples of dimensions (6x2.54 cm).
2. If present release paper is removed from the samples.

Test operation:

1. Tensile tester setting
2. Place the samples between the clamps placing them symmetrically with to the clamps.
3. Run the test on a minimum of 3 samples.
4. Report the average force of 3 tests in N (Newton).

Peel adhesion method

This is a quantitative method to determine the average peel force required to remove a skin at a specified peel angle and speed.

Equipment

| Scissors | Convenient source |
|---|---|
| Standard ruler | Convenient source |
| Steel Roller | 5.0kg Mass. 13cm in diameter and 4.5cm in width covered with 0.5mm thick rubber. |
| Polyester Film | PET 23µ available from EFFEGIDI S.p.A.,43052 Colorno, Italy. |
| Transfer Adhesive | 3M 1524 available from 3M Italia S.p.a. ,20090 Segrate Italy |
| Stop watch | Convenient source |
| Tensile Tester | Instron mod.: 6021 ( or equivalent) |

Test procedure

A) Tensile Tester Peel Settings:

| Load cell | 10N |
|---|---|
| Test Speed | 1000 mm/min |
| Clamp to Clamp distance | 25 mm |
| Pre Loading | 0.2N |
| Test Path "LM" | 50mm |
| Measure variable | F average (N) in "LM" |

B) Skin Condition and Preparation
The sample is peeled from the forearm. There are 3 conditions of the skin that are tested:

1) Dry: The forearm is untreated and not wiped prior to test or between repetitions.

2) Wet: To one cotton disk (Demak'up diameter 5.5cm, weight about 0.6g), 3ml of distilled water is added. Next the disk is then wiped with a light pressure 3 times over the test area on the forearm. (The test area of the forearm is a rectangle approximately 2cm wider and longer than the adhesive area).

C) Sample preparation

1. Allow the samples to adjust to conditioned room ($23 \pm 2°$ Celsius and $50\pm2\%$RH) for about 1 hr.

2. Prepare rectangular adhesive samples 260mm $\pm2$ length and 20mm $\pm2$ wide.

3. Attach on the sample surface the polyester film (using the transfer adhesive to attach the polyester to the substrate surface).

4. Each test specimen should be prepared individually and tested immediately.

5. Remove the release paper from the adhesive without touching it. Attach one end to the skin (see section B).

6. Roll the Steel Roller for 160mm along the adhesive strip, once in each direction.

D) Test Environment

There are 2 environments the adhesive can be tested in:

1) Conditioned Room as described in C1.

2) Wet Environment. Here, after step C4, the specimen is taken and put in a humidity controlled oven for 3 hours at 85degC. It is then taken out and steps C5, C6 are carried out.

E) Execution

1 minute after Step C6, take the free end of the specimen (approx. 100mm long) and insert it in the upper end of the adhesion testing machine. Ensure the specimen is at a 90 degree angle to the forearm. Start the testing machine.

F) Report

Report the average of the peel strength of 5 tests. The single values are the base to calculate the standard deviation between the samples.

**Maximum water absorption/transport rate and capacity test method**

Principle

This test determines the following Performance parameters for an hydrogel adhesive that is laterally confined in a piston/cylinder apparatus:

1. Maximum Absorption/Transport Rate (in units of $gm/cm^2/sec^{0.5}$)

2. Capacity (in units of gms water/gm hydrogel adhesive at specified times)

The test fluid for the test is water. This fluid is absorbed by the hydrogel adhesive under demand absorption conditions at atmosphere pressure. The hydrogel adhesive is tested at a specified relatively-high basis weight. A piston/cylinder apparatus is used to laterally confine the hydrogel adhesive and impose a specified static confining pressure. The bottom of the cylinder is faced with a No. 400 mesh screen to retain the hydrogel adhesive and permit absorption and z-direction transport of water. A computer-interfaced sorption apparatus is used to measure the water absorption versus time.

Equipment

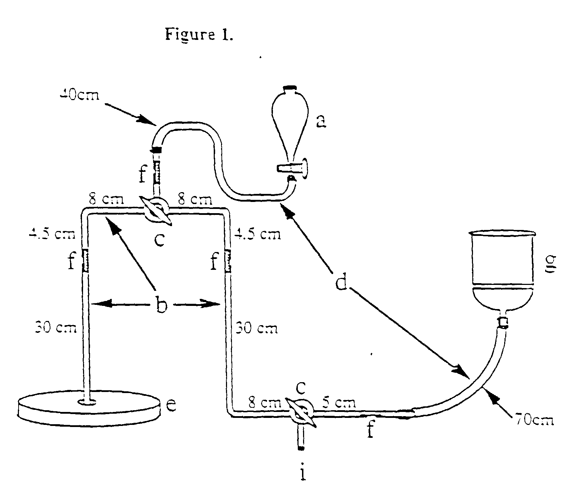

Sorption Apparatus with computer        See Figure 1

a) 500 ml Separatory Funnel - filling reservoir
b) Glass tubing, 2 mm ID, 8 mm OD
c) Glass, 3-way Stopcock, 2 mm ID minimum
d) Tygon Tubing, 1/4" ID, 3/8" OD
e) 150 mm x 15 mm Petri Dish - reservoir
f) Teflon Inserts with o-rings, 2 mm - glass tubing connectors
g) Course Fritted Buchner Funnel, 8 cm Diameter, 8 cm Depth
i) Drain

Cylinder/Piston Apparatus:

| Supplier: | Thiemann Engineering and Manufacturing Company P.O. Box 53126 Cincinnati, Ohio 45253 |
|---|---|
| Balance, 4 decimal places | Mettler AE163 |
| Erlenmeyer Flask, 6000mL | Kimble #26500-6000 |
| Stir Plate, 18 cm x 18 cm | Fisher Stirring Hot Plate #310T |
| Stirring Bar, Teflon, 7.5 cm | Fisherbrand #14-511-85 |
| Weighing Paper, 100 cm x 100 cm | Fisherbrand #09-898-12B |
| Scissors | Conventional source |

**Procedure**

Preparation of the Apparatus

Once set-up is complete, prepare for each measurement by doing the following:

1. Forward flush the fritted disc with water so that it is filled with fresh fluid. Pour off the excess.

2. Cover the funnel.

3. Equilibrate the reservoir and the frit by opening the connecting valves.

4. Close all valves.

5. Drain the frit for 5 minutes by opening the 3-way valve to the drain.

6. Close the drain valve.

The quantity of fluid that drains from the frit in this procedure (called the frit correction weight) is measured by conducting the experiment (see below) for a time period of 15 minutes <u>without</u> the piston/cylinder apparatus. Essentially all of the fluid drained from the frit via this procedure is very quickly reabsorbed by the frit when the experiment is initiated. Thus, it is necessary to substract this frit-correction weight from weights of fluid removed from the reservoir during the test (see below). The frit correction is stable so this procedure only needs to be performed every 10 - 15 measurements.

Before weighing the hydrogel adhesive, wipe the inner surface of the cylinder and outer surface of the cup with isopropanol. Allow to air dry. Weigh approximately 0.9 gms ($\pm$ 0.005 gm) of the hydrogel adhesive onto weighing paper. Record the exact weight (to +/- 0.001 g). Put the hydrogel adhesive into the cylinder and spread evenly on the screen by gently shaking and/or tapping. Take care to assure the hydrogel adhesive is not adhering to the cylinder wall. Place the funnel cover on the fritted funnel. Check the balance for stability (i.e. weight is constant, no change in the third decimal place). Initiate the measurement by opening the 3-way valve between the sample and the reservoir. With auto-initiation, data collection starts immediately, as the fritted disc begins to reabsorb fluid. Data is recorded for at least 60 minutes. The computer program which acquires the data should take readings more frequently the first 10 minutes when fluid is being absorbed most rapidly.

A single measurement is normally sufficient as long as a smooth curve is obtained.

Calculations

Capacity is reported in units of gms water/gm hydrogel adhesive. Capacity at any time is determined as follows:

$$\text{Capacity (t)} = \frac{\{W_r(t=0) - W_r(t) - W_{fc}\}}{W_{\text{hydrogel adhesive: wet basis}}}$$

Where t is the elapsed time from initiation, $W_r(t=0)$ is the weight in grams of the reservoir prior to initiation,

$W_r(t)$ is the weight in grams of the reservoir at elapsed time t, $W_{fc}$ is the frit correction weight in grams (measured separately), and $W_{hydrogel\ adhesive:\ wet\ basis}$ is the weight in grams of the hydrogel adhesive. Capacity is typically reported for times of 5, 10, 30, and 60 minutes after initiation.

The Absorption/Transport Rate is reported in units of $gm/cm^2/sec^{0.5}$. The Rate at any time is determined as follows:

$$\text{The Absorption/Transport Rate (t)} = (1/A_c) * \frac{\{W_r(t) - W_r(t + dt)\}}{\{(t+dt)^{0.5} - (t)^{0.5}\}}$$

where t is elapsed time in units of seconds, $W_r(t)$ is the weight in grams of the reservoir at elapsed time t, $W_r(t+dt)$ is the weight in grams of the reservoir at elapsed time t+dt, and $A_c$ is the area of the hydrogel in units of $cm^2$. If determined graphically, the Uptake Rate is the tangent to the smoothed uptake curve where the y-axis is fluid uptake in units of $gm/cm^2$ and the x-axis is the square root of time, where time is in units of seconds.

**Claims**

1. A wound dressing comprising a hydrogel adhesive wherein said wound dressing has

    a) a moisture vapour transport rate of from $100g/(m^2 24hrs.)$ to $2000g/(m^2 24hrs.)$

    b) a water absorption capacity of at least 100% by weight

    c) a water absorption/transport rate of at least $0.0001g/cm^2/s^{1/2}$,

    d) a peel force of from 0.5N/inch to 6N/inch

    e) a flexibility of less than 0.22N

    f) a tensile strength at 50% elongation of less than 25N and

    g) wherein said adhesive is provided as a layer having a thickness C measured in millimetres (mm), said adhesive having a viscous modulus at a temperature of 25°C (77°F), $G''_{25}$, wherein said viscous modulus $G''_{25}$ (100 rad/sec) and said thickness C of said adhesive satisfy the following equation:

$$G''_{25} \leq [(7.00 + C) \times 3000] \text{ Pa}$$

2. A wound dressing according to claim 1 characterised in that said viscous modulus $G''_{25}$ (100 rad/sec) and said thickness C satisfy the following equation:

$$G''_{25} \leq [(5.50 + C) \times 1700] \text{ Pa}$$

3. A wound dressing according to claim 1 or 2, characterised in that said hydrogel adhesive has an elastic modulus at a temperature of 37°C (100°F), $G'_{37}$, and having a viscous modulus at a temperature of 37°C (100°F), $G''_{37}$, and is selected so that;

    a) $G'_{37}$ (1 rad/sec) is in the range 500 Pa to 20000 Pa, preferably 700 Pa to 15000 Pa, most preferably 1000 Pa to 10000 Pa;

    b) $G''_{37}$ (1 rad/sec) is in the range 100 Pa to 15000 Pa, preferably 100 Pa to 10000 Pa, most preferably 300 Pa to 5000 Pa; and

    c) the ratio $G'_{37}$ (1 rad/sec) / $G''_{37}$ (1 rad/sec) is in the range 1 to 30.

4. A wound dressing according to any one of the preceding clams, wherein said wound dressing has a vapour permeability of from 200g/m²24hrs., to 1500g/m²/24hrs., preferably from 200g/m²/24hrs to 1000g/m²/24hrs.

5. A wound dressing according to any one of the preceding clams, wherein said wound dressing has a peel force of 1.0N/inch to 3.5N/inch, preferably from 1.0N/inch to 2.5N/inch.

6. A wound dressing according to any one of the preceding claims, wherein said wound dressing has a moisture absorption capacity of at least 300% by weight, preferably at least 500% by weight.

7. A wound dressing according to any one of the preceding claims wherein said wound dressing has a water absorption/transport rate of from $0.0005g/cm^2/s^{1/2}$ to $0.05g/cm^2/s^{1/2}$, , preferably from $0.0075g/cm^2/s^{1/2}$ to $0.035g/cm^2/s^{1/2}$.

8. A wound dressing according to any one of the preceding, wherein said wound dressing has a flexibility of less than 0.012N, preferably less than 0.0016N.

9. A wound dressing according to any one of the preceding claims wherein said wound dressing has a tensile strength at 50% elongation of less than 10N.

10. A wound dressing according to any one of the preceding claims, wherein said hydrogel adhesive is a three dimensional gel matrix comprising a polymer selected from acrylics, sulphonated polymers, vinyl alcohols, vinyl pyrrolidine, polyethylene oxide or mixtures thereof, and a plasticiser selected from polyhydric alcohols, polyethylene glycols, glycerol, sorbitol, water or mixtures thereof.

11. A wound dressing according to any one of the preceding claims, wherein said dressing further comprises a supportive substrate.

12. A wound dressing according to any one of the preceding claims, wherein said wound dressing comprises from 20 $g/m^2$ to $2500g/m^2$ of said hydrogel adhesive.

Figure 1.

**European Patent Office**

## PARTIAL EUROPEAN SEARCH REPORT

which under Rule 45 of the European Patent Convention shall be considered, for the purposes of subsequent proceedings, as the European search report

Application Number

EP 99 12 2204

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 5 369 155 A (ASMUS ROBERT A) 29 November 1994 (1994-11-29) * abstract * * column 1, line 14-36 * * column 2, line 34-56 * * column 5, line 1-49 * * column 6, line 5-42,61-66 * * column 7 - column 9 * * column 17, line 14-18; table 1 * --- | 1-12 | A61L15/58 A61L15/60 |
| X | US 5 674 275 A (TANG JINSONG ET AL) 7 October 1997 (1997-10-07) * abstract * * column 2 * * column 5, line 22-28 * * column 9, line 51-60 * * column 10, line 62-66 * * column 11, line 64 - column 12, line 24; tables 7,8 * --- -/-- | 1-12 | |

TECHNICAL FIELDS SEARCHED (Int.Cl.7)

A61L

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| THE HAGUE | 22 August 2000 | Böhm, I |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

**European Patent Office**

**INCOMPLETE SEARCH SHEET C**

**Application Number**

EP 99 12 2204

Claim(s) searched completely:
       10-12

Claim(s) searched incompletely:
       1-9

Reason for the limitation of the search:

Present claims 1-9 relate to a product defined (inter alia)

by reference to the following parameter(s):

       P1: the quotient : water absorption/water transport rate (WTR)
       P2: peel force 'N/inch!
       P3: flexibility 'N!
       P4: tensile strength elongation 'N!
       equation I: G'' (25)=< '(7.00 + C) * 3000! Pa
       equation I: G'' (25)=< '(5.50 + C) * 1700! Pa

       with C 'mm!, G''(25) '100rad/sec! ; G' : elastic modulus ;  G'' : viscous modulus

The use of these parameters in the present context is considered to lead to a lack of clarity within the meaning of Article 84 EPC. It is impossible to compare the parameters the applicant has chosen to employ with what is set out in the prior art. The lack of clarity is such as to render a meaningful complete search impossible. Consequently, the search of claims 1-9 has been restricted to those adhesive hydrogels in which the polymers are these according to claim 10.

European Patent
Office

**PARTIAL EUROPEAN SEARCH REPORT**

Application Number

EP 99 12 2204

| | DOCUMENTS CONSIDERED TO BE RELEVANT | | CLASSIFICATION OF THE APPLICATION (Int.Cl.7) |
|---|---|---|---|
| Category | Citation of document with indication, where appropriate, of relevant passages | Relevant to claim | |
| X | US 5 846 214 A (ISOBE KAZUKI ET AL) 8 December 1998 (1998-12-08) * abstract * * column 1, line 7-14 * * column 2, line 3-36 * * column 3, line 16-34 * * column 5, line 31-37 * * claim 12; example 5 * --- | 1-12 | |
| X | EP 0 852 149 A (PROCTER & GAMBLE) 8 July 1998 (1998-07-08) * abstract * * page 2, line 5-24 * * page 3, line 11-57 * * page 4, line 25-57 * * page 6, line 46 - page 7, line 14 * * claim 1 * ----- | 1-10,12 | TECHNICAL FIELDS SEARCHED (Int.Cl.7) |

EPO FORM 1503 03.82 (P04C10)

**ANNEX TO THE EUROPEAN SEARCH REPORT
ON EUROPEAN PATENT APPLICATION NO.**

EP 99 12 2204

This annex lists the patent family members relating to the patent documents cited in the above-mentioned European search report.
The members are as contained in the European Patent Office EDP file on
The European Patent Office is in no way liable for these particulars which are merely given for the purpose of information.

22-08-2000

| Patent document cited in search report | | Publication date | Patent family member(s) | | Publication date |
|---|---|---|---|---|---|
| US 5369155 | A | 29-11-1994 | US | 5270358 A | 14-12-1993 |
| | | | AT | 120967 T | 15-04-1995 |
| | | | AU | 648536 B | 28-04-1994 |
| | | | AU | 7074791 A | 24-07-1991 |
| | | | BR | 9007960 A | 27-10-1992 |
| | | | CA | 2071004 A | 29-06-1991 |
| | | | DE | 69018650 D | 18-05-1995 |
| | | | DE | 69018650 T | 14-12-1995 |
| | | | DK | 507878 T | 31-07-1995 |
| | | | EP | 0507878 A | 14-10-1992 |
| | | | ES | 2071297 T | 16-06-1995 |
| | | | FI | 922986 A | 26-06-1992 |
| | | | HK | 91196 A | 31-05-1996 |
| | | | KR | 147306 B | 01-08-1998 |
| | | | NO | 922553 A | 26-08-1992 |
| | | | WO | 9109633 A | 11-07-1991 |
| US 5674275 | A | 07-10-1997 | US | 5614586 A | 25-03-1997 |
| | | | CA | 2146286 A | 07-10-1995 |
| | | | DE | 69505176 D | 12-11-1998 |
| | | | DE | 69505176 T | 10-06-1999 |
| | | | EP | 0676457 A | 11-10-1995 |
| US 5846214 | A | 08-12-1998 | JP | 9262279 A | 07-10-1997 |
| | | | JP | 9267453 A | 14-10-1997 |
| | | | JP | 9262249 A | 07-10-1997 |
| EP 0852149 | A | 08-07-1998 | EP | 0850619 A | 01-07-1998 |
| | | | EP | 0853934 A | 22-07-1998 |
| | | | AU | 5612998 A | 17-07-1998 |
| | | | AU | 5619798 A | 17-07-1998 |
| | | | AU | 5709198 A | 17-07-1998 |
| | | | AU | 5709498 A | 17-07-1998 |
| | | | CN | 1246060 A | 01-03-2000 |
| | | | CN | 1246061 A | 01-03-2000 |
| | | | CN | 1246062 A | 01-03-2000 |
| | | | CN | 1246064 A | 01-03-2000 |
| | | | EP | 0968007 A | 05-01-2000 |
| | | | EP | 0946215 A | 06-10-1999 |
| | | | EP | 0948366 A | 13-10-1999 |
| | | | EP | 0948367 A | 13-10-1999 |
| | | | WO | 9828016 A | 02-07-1998 |
| | | | WO | 9828020 A | 02-07-1998 |
| | | | WO | 9828021 A | 02-07-1998 |
| | | | WO | 9828024 A | 02-07-1998 |
| | | | AU | 5385398 A | 17-07-1998 |

EPO FORM P0459

For more details about this annex : see Official Journal of the European Patent Office, No. 12/82

## EP 1 051 983 A1

### ANNEX TO THE EUROPEAN SEARCH REPORT
### ON EUROPEAN PATENT APPLICATION NO.

EP 99 12 2204

22-08-2000

| Patent document cited in search report | Publication date | Patent family member(s) | Publication date |
|---|---|---|---|
| EP 0852149 A | | AU 5531598 A | 17-07-1998 |
| | | AU 5612598 A | 17-07-1998 |
| | | AU 5612698 A | 17-07-1998 |
| | | AU 5619598 A | 17-07-1998 |
| | | AU 5619698 A | 17-07-1998 |
| | | AU 5708898 A | 17-07-1998 |
| | | AU 5801498 A | 17-07-1998 |
| | | AU 5801598 A | 17-07-1998 |
| | | CN 1246044 A | 01-03-2000 |
| | | CN 1246063 A | 01-03-2000 |
| | | CN 1246065 A | 01-03-2000 |
| | | CN 1246066 A | 01-03-2000 |
| | | CN 1246067 A | 01-03-2000 |
| | | CN 1246068 A | 01-03-2000 |
| | | EP 0850624 A | 01-07-1998 |
| | | EP 0850628 A | 01-07-1998 |
| | | EP 0850649 A | 01-07-1998 |
| | | EP 0855190 A | 29-07-1998 |
| | | EP 0948364 A | 13-10-1999 |
| | | EP 0957871 A | 24-11-1999 |
| | | EP 0948365 A | 13-10-1999 |
| | | EP 1023019 A | 02-08-2000 |
| | | EP 0946213 A | 06-10-1999 |
| | | EP 0946214 A | 06-10-1999 |
| | | EP 1006973 A | 14-06-2000 |
| | | EP 0948368 A | 13-10-1999 |
| | | EP 0948369 A | 13-10-1999 |
| | | JP 2000505701 T | 16-05-2000 |
| | | JP 2000505703 T | 16-05-2000 |
| | | NO 993054 A | 23-08-1999 |
| | | NO 993060 A | 23-08-1999 |

EPO FORM P0459